# EUROPEAN PATENT SPECIFICATION

(11) **EP 3 000 474 B1**
(45) Date of publication and mention of the grant of the patent: **28.11.2018**
(21) Application number: 14801762.7
(22) Date of filing: 20.05.2014
(51) Int. Cl.: A61K 9/107, A61K 47/44, A61K 47/26, A61K 47/10, A61K 47/14, A61K 38/13, A61P 27/02

(54) **NANOEMULSION EYEDROP COMPOSITION CONTAINING CYCLOSPORINE AND METHOD FOR PREPARING SAME**
NANOEMULSIONS-AUGENTROPFENZUSAMMENSETZUNG MIT CYCLOSPORIN UND VERFAHREN ZUR HERSTELLUNG DAVON
COMPOSITION DE GOUTTE OCULAIRE DE NANO-ÉMULSION CONTENANT DE LA CYCLOSPORINE ET SON PROCÉDÉ DE PRÉPARATION

(30) Priority: 20.05.2013 KR 20130056561; 13.03.2014 KR 20140029939
(43) Date of publication of application: 30.03.2016
(73) Proprietor: Taejoon Pharm. Co., Ltd., Seoul 140-887 (KR)
(72) Inventor: LEE, Joon Youb, Seoul 140-887 (KR); SHIN, Youn Jae, Seoul 136-110 (KR); RYU, Sang-Rok, Suwon-si Gyeonggi-do 442-816 (KR)
(74) Representative: D Young & Co LLP
(86) International application number: PCT/KR2014/004489
(87) International publication number: WO 2014/189251

(56) References cited:
- WO-A2-2012/091278
- WO-A2-2012/091278
- CN-A- 101 244 256
- US-A1- 2006 205 639
- US-A1- 2006 205 639
- US-A1- 2007 027 072
- US-A1- 2012 093 894

## Description

### [Technical Field]

The present disclosure relates to an ophthalmic nanoemulsion composition and a method for preparing the same which increase the solubility of cyclosporine as an active ingredient and improves the stability of the ophthalmic composition by mixing cyclosporine, a nonaqueous solvent, an emulsifier, and an aqueous solvent, and use of the composition in a method for preventing or treating ocular disease.

### [Background Art]

An immunosuppressive drug is a medicine used for immunosuppressive therapy by preventing or inhibiting abnormal immunoreactivity. The immunosuppressive drug is currently being used as a therapeutic agent for various diseases including transplant rejection after the organ and tissue transplantation; inflammatory intestinal disease such as ulcerative colitis or Crohn's disease; rheumatoid arthritis; Behcets syndrome; inflammatory or allergic dermatosis such as psoriasis or atopic dermatitis; inflammatory or allergic respiratory disease such as chronic obstructive pulmonary disease or Asthma; systemic lupus erythematosus; scleroderma; Sjogren syndrome; and dry eye syndrome among others.

Sjogren syndrome is a chronic inflammatory disease of exocrine gland and specifically, it is characterized in decrement of generation of saliva and tear *via* destruction of normal tissues of salivary gland and lacrimal gland. The cause of Sjogren syndrome is not completely revealed but genetic factors such as family history, viruses, cytokines and autoimmune antibodies are reported as the causes of Sjogren syndrome. Currently, cyclosporine which is an immunosuppressive drug, is used for treating Sjogren syndrome, and nonsteroidal anti-inflammatory drugs or steroids are used along with cyclosporine when symptom is severe.

Dry eye syndrome or immune keratoconjunctivitis sicca (KCS) patients generally complain of soreness, dryness, foreign body sensation and stinging sensation in the eyes. Additionally, it is uneasy to open eyes because eyes are easily being fatigue, and thus feel comfortable when the eyes are closed and symptoms being severe when the eyes are open. The eyes were slightly bloodshot in outward appearance and patients complain headache when symptom is severe. This dry eye syndrome occurs due to tear deficiency caused by insufficient generation and excessive evaporation of tears, imbalance of components of tears, or inflammation of the eye or damage on the intraocular epithelial cells. Within current drugs, a dry eye syndrome supplement (e.g. artificial tears) that temporarily improves dryness and foreign body sensation and cyclosporine as a therapeutic agent that increases tear secretion through immunosuppressive effect in the dry eye are representative.

Cyclosporines are polypeptides consisted of 11 amino acids, and exhibit powerful immunosuppressing activity by inhibiting proliferation and differentiation of T-cell. US Patent No. 4,839,342 discloses cyclosporine's immunosuppressing activity as well as disclosing that cyclosporines are effective drugs in treating immune keratoconjunctivitis sicca (KCS).

Sirolimus, tacrolimus and its derivatives other than cyclosporines are known as ophthalmic preparations.

Cyclosporines have cyclic structure comprising seven N-methylated amino acids and four non-N-methylated amino acids, and there are cyclosporine A, cyclosporine B, cyclosporine C, cyclosporine D and cyclosporine G among others according to the structure of the constituting amino acid residue, and practically, cyclosporine A, of which pharmacological activity and clinical cases are revealed more than the rest, is studied most widely. Intramolecular attraction of cyclosporines is strong and interaction with water molecule is relatively difficult, and thus cyclosporines are poorly water-soluble drugs that are hardly dissolved in water. Water solubility of cyclosporines is known as about 20 µ g/ml to 30 µ g/ml, and it is very difficult to prepare a water-soluble medicament composition with cyclosporine having such low water solubility.

Restasis™, currently being sold as a cyclosporine ophthalmic composition, is a milky-colored opaque emulsion and has demerits of inducement of burning sensation accompanied by conjunctival injection, pruritus, blurred vision and foreign body sensation when it is administered to the eyes. Therefore, a purpose of designing emulsion-type ophthalmic preparation containing the poorly water-soluble cyclosporine as an active ingredient is to stably improve the water solubility of cyclosporine and to minimize the unpleasant symptoms of ophthalmic administration by improving irritation, foreign body sensation, burning sensation, soreness, hyperemia, blurred vision and pruritus.

Existing ophthalmic emulsions generally contain 2 or more immiscible ingredients in a single composition, and thus it is common to form 2 separate phases in a composition.

Thermodynamically, the emulsion is in unstable status and tends to separate into various phases through pathways such as flocculation, sedimentation, creaming, ostwald ripening and coalescence among others. Researches on nanoemulsion wherein its particle size is reduced to nano-size have been actively conducted to resolve the instability of the emulsion. In terms of a preparation process, the known emulsions such as the Restasis™ are prepared by using a high speed agitator-type or a high speed shear-type apparatus such as a high pressure homogenizer or a microfluidizer which delivers great physical force to compositions while preparing the emulsion. As disclosed in the Korean Patent Publication No. 10-2008-0030828, this preparation process requires large manufacturing facility and heavy expenditure, and hardly applicable to heat-sensitive ingredients due to substantial temperature elevation caused by energies delivered to the emulsion while manufacturing. Also, cyclosporine emulsion prepared by this process was quickly flocculated due to unequal oil-drop size, and thus creaming process is accelerated and affects the long-term stability. In addition, there is a difficulty in securing uniform quality for each manufacturing lot because particle size distribution in a dispersed phase is relatively wide.

The documents US 2007/027072 and CN 101 244 256 disclose cyclosporine ophthalmic formulations.

### [Prior Art Reference]

### [Patent Reference]

US Patent No. 5,660,858
International Publication No. WO1995/31211
Korean Patent No. 10-1008189
US Patent No. 2007/027072
Chinese Patent No. CN 101 244 256

### [Summary of Invention]

### [Technical Problem]

The inventors of the present disclosure confirmed that solubility of cyclosporine is increased, average particle size is formed in range of 1 nm to 100 nm and maximum particle size is formed in 220 nm or less, and the penetrance and efficacy can be improved and physicochemical stability, irritation, blurred vision and foreign body sensation can be effectively improved when ophthalmically administering the final product of nanoemulsion in case of preparing the ophthalmic nanoemulsion by appropriately mixing ingredients of the nanoemulsion while conducting a research on the ophthalmic nanoemulsion which can improve solubility of cyclosporine as an active ingredient, and accordingly completed the present disclosure.

Therefore, the present invention provides an ophthalmic nanoemulsion composition comprising:
- cyclosporine in the amount of 0.02 - 0.3 w/v% based on the total amount of the composition;
- castor oil in the amount of 8 times or more of the amount of the cyclosporine to 2.5 w/v% or less based on the total amount of the composition;
- one or more hydrophilic emulsifiers selected from a group consisting of polyoxyl 35 castor oil, polyoxyethylene sorbitan fatty acid esters and polyoxyethylene fatty acid esters;
- one or more hydrophobic emulsifiers selected from a group consisting of sorbitan fatty acid esters, glycerin fatty acid esters, diethylene glycol monoethyl ether, polyethylene glycols, propylene glycol and propylene glycol esters of fatty acids; and
- an aqueous solvent.

The present disclosure also provides ophthalmic nanoemulsion compositions having average particle size range of 1 nm to 100 nm.

The present disclosure also provides the ophthalmic nanoemulsion for use in a method of preventing or treating ophthalmic diseases.

### [Solution to Problem]

The present disclosure provides ophthalmic nanoemulsion as defined in the claims.

The ophthalmic nanoemulsion compositions of the present disclosure can be prepared by appropriately mixing the ingredients, and sterile filtration is available, solubility of cyclosporine is increased and stability is improved because its average particle(globule) size is formed in 200 nm or less or within the range of 1 nm to 100 nm and particle size distribution is narrow.

'Cyclosporine' is an active ingredient of the ophthalmic nanoemulsion compositions and can include cyclosporine A, cyclosporine A derivatives, cyclosporine B, cyclosporine C, cyclosporine D or its mixtures thereof among others, and preferably cyclosporine can be cyclosporine A or its derivatives thereof.

Cyclosporine can be contained in a therapeutically effective amount to improve dry eye syndrome and is contained in 0.02 to 0.3w/v% based on the total ophthalmic nanoemulsion compositions for the purposes of the present disclosure.

With regard to preparation of an ophthalmic preparation, a composition wherein surfactant such as polyoxyethylated castor oils and polyoxyethylene sorbitan fatty acid esters among others is used for preventing precipitation of cyclosporine in the eyes after administration of a eye drop is disclosed, but its demerits are also known that the ophthalmic composition would exist in status of milky-colored opaque emulsion when using this composition and accordingly inducing blurred vision at initial stage of administration. Therefore, the ophthalmic nanoemulsion compositions are prepared through the present disclosure by properly selecting the nonaqueous solvent, the hydrophilic emulsifier and the hydrophobic emulsifier to resolve the above problems.

The 'nonaqueous solvent' is castor oil and it is commercially available under product name of castor oil (manufactured by ITHO oil chem., Japan). Castor oil decreases tear evaporation at the ocular surface and has superior spreadability compared to other oils, and thus castor oil is useful for treating dry eye syndrome such as a meibomian gland dysfunction at the lacrimal gland among others. However, the ophthalmic composition comprising the nonaqueous solvent such as castor oil among others might induce pains including an ophthalmic irritation, and visual disturbance. Therefore, it is desirable to use the nonaqueous solvent (i.e. castor oil among others) for the present disclosure in minimum concentration by which cyclosporine can be dissolve properly and adverse reactions can be minimized. By using the nonaqueous solvent in minimum concentration, the amount of the emulsifier can also be minimized which is used for stabilizing an oil phase, therefore it is available to provide the safer ophthalmic nanoemulsion compositions compared to cyclosporine emulsions on the market. The amount of the nonaqueous solvent can be 0.01 - 10.0 w/v%, 0.1 - 5.0 w/v %, and preferably 0.1 - 2.5 w/v % based on the total amount of the composition.

The amount of castor oil can be 8 times or more of the amount of cyclosporine as active ingredient to 2.5 w/v % or less based on the total amount of the composition. The most stable nanoemulsion composition is formed when the amount of castor oil (i.e. the nonaqueous solvent) is 8 times or more of the amount of cyclosporine to 2.5 w/v % or less based on the total amount of the composition, therefore this amount is desirable to prepare the stable nanoemulsion composition of the present disclosure.

The nanoemulsion compositions of the present disclosure comprise one or more hydrophilic and one or more hydrophobic emulsifiers that help emulsification of the nonaqueous solvent in the aqueous solvent. One or more emulsifiers can be selected by considering ratio of Hydrophilic-Lipophilic Balance (hereinafter, HLB) values of each emulsifier depending on a required HLB value of the nonaqueous solvent, preferably depending on the required HLB value of castor oil. One or more emulsifiers can be selected from a group of the hydrophilic emulsifiers in which the HLB value is at least 8, specifically 10 or more, and from a group of the hydrophobic emulsifiers in which the HLB value is less than 8, specifically 6 or less.

The hydrophilic emulsifier is selected from the group consisting of polyoxyl 35 castor oil, polyoxyethylene sorbitan fatty acid ester and polyoxyethylene fatty acid ester. Polyoxyl 35 castor oil and the preferred polyoxyethylene sorbitan fatty acid ester, polyoxyethylene sorbitan monooleate, are both being sold in the market under the product name of Cremophor EL™ or ELP™ (BASF), and Polysorbate 80 (NOF corporation). The amount of the hydrophilic emulsifier can be 0.01 - 10.0 w/v%, 0.01 - 7.0 w/v% based on the total amount of the composition and most preferably 0.1 - 5.0 w/v%.

The amount of polyoxyl 35 castor oil is preferably at least 1.6 times or more of the amount of castor oil (i.e. the nonaqueous solvent) to 5.0 w/v% or less based on the total amount of the composition. Also, the amount of polyoxyl 35 castor oil is desirable to be at least 12.8 times of the amount of cyclosporine (i.e. active ingredient) to 5.0 w/v% based on the total amount of the composition. A stable nanoemulsion having average particle size of 1 nm to 100 nm can be easily prepared with the contents stated above and show the best sensation by ocular instillation because the hydrophilic emulsifier is contained in 5.0 w/v% or less based on the total amount of the composition.

The hydrophobic emulsifier is selected from the group consisting of sorbitan fatty acid esters, glycerin fatty acid esters, diethylene glycol monoethyl ether, polyethylene glycols, propylene glycols and propylene glycol esters of fatty acids. Specifically, the preferable hydrophobic emulsifiers are polyethylene glycols (PEG), propylene glycol, sorbitan fatty acid esters and diethylene glycol monoethyl ether, and these are being sold on the market as product names: Super Refined PEG 300™, Super Refined PEG 400™, Super Refined PEG 600™(Croda), Propylene Glycol(Merck), Span 20, Span 80(Croda) and Transcutol P(Gattefosse), respectively. The amount of said hydrophobic emulsifier can be 0.01 - 7.0 w/v% based on the total amount of the composition, and preferably it can be 0.1 - 5.0 w/v% based on the total amount of the composition. Additionally, the hydrophobic emulsifier can be polyethylene glycol, propylene glycol and diethylene glycol monoethyl ether, and the amount of these can be at least 0.1 w/v% or more based on the total amount of the composition, and it is preferable to be contained in 3 times or less than the amount of the hydrophilic emulsifier, which would preferably be polyoxyethylene hydrogenated castor oil. Also, in this case, it is most preferable that does not exceed 5.0 w/v% based on the total amount of the composition. It is most preferable that the hydrophobic emulsifier is contained at least 0.1 w/v% or more based on the total amount of the composition to prepare a stable nanoemulsion composition, and it is preferable that the hydrophobic emulsifier is contained 3 times or less than the amount of the hydrophilic emulsifier, preferably polyoxyethylene hydrogenated castor oil and 5.0 w/v% or less based on the total amount of the composition in order to achieve superior sensation by ocular instillation.

The aqueous solvent of the present disclosure is an adequate ingredient for preparation of ophthalmic preparations, and it can be sterile purified water, saline solution and water for injection.

In addition, the present disclosure can further comprise a stabilizer in the ophthalmic nanoemulsion composition. Physicochemical stabilities of the ophthalmic nanoemulsion composition of the present disclosure can be improved more with the additional inclusion of the stabilizer. The stabilizer can provide viscosity through gridding oil-drop of the nanoemulsion by forming a certain bonding structure *via* hydration in the aqueous solvent, and can act as physically stabilizing the nanoemulsion. The stabilizer can comprise cellulose-based compounds including carboxymethyl cellulose(CMC), hydroxypropyl methylcellulse(HPMC) and hydroxyethylcellulose(HEC) among others; polyvinyl-based compounds including polyvinyl alcohol(PVA) and polyvinylpyrrolidone(PVP) among others; acrylic-based compounds including carbomer among others; gum-based compounds including gellan gum and xanthan gum among others; polysaccharides including hyaluronic acid(HA), sodium hyaluronate, sodium alginate and dextran among others; or its random combinations thereof. Additionally, the stabilizer can be at least one selected from a group consisting of carboxymethyl cellulose, xanthan gum, hyaluronic acid(HA) and sodium hyaluronate.

Therefore, the composition of the present disclosure can comprise cyclosporine, the nonaqueous solvent, the hydrophilic emulsifier, the hydrophobic emulsifier, the stabilizer and the aqueous solvent.

The amount of the stabilizer can be 0.001 - 10.0 w/v%, 0.01 - 5.0 w/v%, and preferably 0.01 - 2.0 w/v% based on the total amount of the composition.

In addition, the ophthalmic nanoemulsion compositions of the present disclosure can further comprise pH modifiers, isotonizing agents, preservatives and buffering agents among others.

The pH adjusting agent can be sodium hydroxide and hydrochloric acid among others, and it can be used to obtain a proper pH value by adding a necessary amount *via* methods known in the art.

The isotonizing agent can be at least one selected from a group consisting of glycerol, mannitol, sorbitol, sodium chloride, potassium chloride, boric acid and borax, and the amount of the isotonizing agent can be in range of 0.01 - 10.0 w/v% based on the total amount of the composition, and can be used in 0.1 - 3.0 w/v%.

The preservative of the present disclosure can be quaternary ammonium compounds including benzalkonium chloride, benzethonium chloride, cetalkonium chloride and polyquaternium-1 (e.g. Polyquad®) among others; guanidine-based compounds including PHMB and chlorohexidine among others; chlorobutanol; mercury-based preservatives including thimerosal, phenyl mercury acetate and phenylmercuric nitrate among others; and oxidative preservatives including stabilized oxychloro complex (e.g. Purite®) and p-oxybenzoic acid alkyls (e.g. p-oxybenzoic acid methyl (PM)) among others.

The buffering agent of the present disclosure can be any buffering agents which are used for eye drop without any restrictions. There are an acetate buffer, a citrate buffer, a phosphate buffer (e.g. sodium phosphate or its hydrate, and sodium dihydrogen phosphate or its hydrate) and a borate buffer such as boric acid or its salt among others but not limited to these. The amount of the buffering agent can be adequately selected by those skilled in the art and it can be added in 0.001 - 10 w/v%, preferably 0.01 - 5.0 w/v%, and more preferably 0.1 - 2.0 w/v% based on the total amount of the composition.

In addition, it is preferable that the particle size of the ophthalmic nanoemulsion compositions of the present disclosure is 220 nm or less, and the particle size in the compositions can be 0 nm to 220 nm, and 0.3 nm to 220 nm.

Additionally, the present disclosure provides an ophthalmic nanoemulsion for use in a method of preventing or treating ophthalmic diseases comprising administration into eye of the ophthalmic nanoemulsion composition to patients.

The ophthalmic diseases can be intrinsic diseases or extrinsic diseases due to external injury or wearing of hard contact lens, and preferably the ophthalmic disease can be Sjogren syndrome or dry eye syndrome, and more preferably, the ophthalmic disease can be dry eye syndrome.

Additionally, the present disclosure provides a method of preparation of the ophthalmic nanoemulsion composition having average particle size of 1 nm - 100 nm comprising: preparing a mixture by stirring cyclosporine, the nonaqueous solvent, the hydrophilic emulsifier, the hydrophobic emulsifier and the aqueous solvent.

Specifically, the present disclosure provides a method of preparation of the ophthalmic nanoemulsion composition having average particle size of 1 nm - 100 nm comprising: preparing the mixture by dissolving cyclosporine (i.e. active ingredient) in the nonaqueous solvent, adding the hydrophilic emulsifier, the hydrophobic emulsifier and the aqueous solvent to the dissolved composition, and stirring the same.

In the above preparation of said mixture, the method of preparation can further comprise additional dissolving of the stabilizer or the isotonizing agent in the aqueous solvent, stirring of the same with the prepared mixture and controlling of its pH scale.

According to the preparation method of the present disclosure, the nanoemulsion having average particle size of 1 nm - 100 nm can be formed because ingredients are adequately mixed; a common sterilizing filtration by using a 0.22 µm filter is available without using an existing high speed stirrer-type or a high speed shear-type apparatus such as a high pressure homogenizer or a microfluidizer because the ophthalmic nanoemulsion composition having a maximum particle size of 220 nm or less is prepared; and the cost of preparing said ophthalmic nanoemulsion composition having a particle size of 220 nm or less is low.

The nanoemulsion composition prepared through the present disclosure has superior effects such as little irritation, foreign body sensation and blurred vision among others, and releases cyclosporine A (i.e. active ingredient) with an appropriate rate at the cellulose membrane-release assay which evaluates release of drugs.

The nanoemulsion composition prepared through the present disclosure can be effectively used for treating dry eye syndrome because patients' inappropriate irritant and foreign body sensation are improved as well as exhibiting high treatment effect in treating dry eye syndrome when using said nanoemulsion composition as an ophthalmic composition for ocular administration, and the nanoemulsion composition would increase tear secretion and retention time of tear film. Also, the amount of the cyclosporine A residues in ocular tissue are expected to be high after administration of the nanoemulsion composition.

### [Advantageous Effect]

The ophthalmic nanoemulsion composition of the present disclosure is having the average particle size of 200 nm or less, preferably 100 nm or less, and the particle distribution is characterized in narrow. Therefore, the ophthalmic nanoemulsion composition of the present disclosure can be effectively used for ophthalmic composition because sterilizing filtration is available, stability is improved and the effect of improving foreign body sensation and blurred vision is clinically superior.

### [Brief Description of Drawings]

Figure 1 illustrates a confirmed particle size distribution of the ophthalmic nanoemulsion composition of Example 33 and Restasis™ eye drop.
Figure 2 illustrates a confirmed distribution stability of the ophthalmic nanoemulsion compositions of Examples 62 and 63, the emulsion and the suspension through Turbiscan Stability Index (TSI).
Figure 3 is a graph showing measured average values of burning sensation and foreign body sensation of Example 62 and the Restasis™ which is a control drug through testing sensation of drop into the eyes.

### [Description of Embodiments]

The present disclosure will be described more fully hereinafter with reference to the accompanying examples. However, the following examples are intended to illustrate the present invention, and the present invention is not limited by the following examples.

### Experimental Example 1. Preparation of the nanoemulsion and measurement of an average particle size of the same in accordance with type and content variation of the nonaqueous solvent

The nanoemulsion compositions were prepared with different amount of castor oil, labrafac lipophile WL 1349 or miglyol 812, and then measured its average particle size. Specific method of preparing the nanoemulsion composition follows. Cyclosporine A and the nonaqueous solvent were mixed in the amount stated in Table 1 below and completely dissolved under 600 - 800 rpm and 70°C by using a stirrer (Super-Nuova™ Multi-place, Thermo Scientific). An oil phase was prepared by adding the hydrophilic and hydrophobic emulsifiers with the contents stated in Table 1 to the above prepared mixed solution and sufficiently mixing via stirring the same. The prepared solution was cooled at room temperature and the oily phase was put into the aqueous solvent for washing the oily phase several times, and then stirred it under 400 - 500 rpm and room temperature by using the stirrer (Super-Nuova™ Multi-place, Thermo Scientific). The aqueous solvent was added until the final volume was to be 100mL after 30 minutes or more of stirring. The nanoemulsion voluntarily formed a stable homogeneous phase through Self Nano-Emulsifying Drug Delivery system (SNEDDS). The size of the particle of the ophthalmic nanoemulsion prepared through the above method was measured by using the Zetasizer(Malvern Instruments, England), which is an apparatus for measuring particle sizes, and the constitution and measured average particle size(nm) of the prepared nanoemulsion composition are shown in Table 1 Examples 4-15 are not in the scope of the claims.

**[Table 1]**

| **Ingredient** | | **Example** | | | | | | | | | | | | | | |
|---|---|---|---|---|---|---|---|---|---|---|---|---|---|---|---|---|
| | | **1** | **2** | **3** | **4** | **5** | **6** | **7** | **8** | **9** | **10** | **11** | **12** | **13** | **14** | **15** |
| **Active Ingredient (w/v%)** | **Cyclosporine** | 0.05 | 0.03 | 0.1 | 0.05 | 0.05 | 0.05 | 0.05 | 0.05 | 0.03 | 0.05 | 0.05 | 0.05 | 0.05 | 0.05 | 0.03 |
| **Nonaqueous Solvent (w/v%)** | **Castor Oil** | 0.42 | 0.25 | 0.84 | | | | | | | | | | | | |
| | **Labrafac** | | | | 2 | 1.8 | 1.8 | 1.8 | 2 | 1.2 | | | | | | |
| | **Miglyol 812** | | | | | | | | | | 0.75 | 0.66 | 0.66 | 0.66 | 0.75 | 0.45 |
| **Hydrophilic Emulsifier (w/v%)** | **Polysorbate80** | | | | | | | | 1.6 | 0.8 | | | | | 1.6 | 0.8 |
| | **Cremorphor ELP** | 1 | 1 | 4.8 | 2.4 | 2.4 | 1.6 | 1.6 | | | 2.4 | 2.4 | 1.6 | 1.6 | | |
| **Hydrophobic Emulsifier (w/v%)** | **Polyethylene Glycol 400** | 0.4 | 0.3 | 0.8 | 0.6 | 0.4 | 0.4 | | 0.4 | 0.2 | 0.6 | 0.4 | 0.4 | | 0.4 | 0.2 |
| | **Propylene Glycol** | 0.3 | 0.2 | 0.6 | | 0.3 | 0.3 | | | | | 0.3 | 0.3 | | | |
| | **Transcutol P** | | | | | | | 0.4 | | | | | | 0.4 | | |
| **Aqueous Solvent** | **Purified Water** | q.s | q.s | q.s | q.s | q.s | q.s | q.s | q.s | q.s | q.s | q.s | q.s | q.s | q.s | q.s |
| | **Average Particle Size** | 41.05 | 31.04 | 35.29 | 45.92 | 48.33 | 58.51 | 54.21 | 76.15 | 73.25 | 30.72 | 32.76 | 42.16 | 44.8 | 62.95 | 60.31 |

All of the particle sizes of the nanoemulsion compositions in Examples 1 to 15 were shown as 220 nm or less in maximum, and as shown in above Table 1, it was confirmed that the average particle size were all 100 nm or less in particular and transparent nanoemulsion compositions were prepared. The nanoemulsion including cyclosporine as a active ingredient can be determined to have a suitable particle size when the maximum particle size is 220 nm or less, and thus it was confirmed that the ophthalmic nanoemulsion composition having a very small average particle size can be prepared according to the compositions of the present disclosure. In particular, Examples 1 to 3 exhibited superior effect in forming the nanoemulsion where the average particle sizes were 41.05, 31.04 and 35.29nm while the examples were consisted as including castor oil having relatively low amount of 0.42, 0.25 and 0.84 w/v% compared to the other nonaqueous solvents (i.e. labrafac and miglyol 812) and accordingly consisted of the emulsifier having low content. When considering that the nonaqueous solvent can give irritation upon administration into eye, castor oil has an advantage that it can minimize the irritation caused by an oil component in ophthalmic compositions, and thus it was confirmed that castor oil is one of the preferred nonaqueous solvent for the ophthalmic nanoemulsion composition.

### Experimental Example 2. Preparation of the nanoemulsion and measurement of an average particle size of the same according to type and varing amount of the emulsifier

The nanoemulsion composition of Examples 16 to 36 were Prepared by the same method explained in Experimental Example 1 *via using* castor oil with the nonaqueous solvent, and varying amount of castor oil and type and amount of the emulsifier. Constitution and amount of the prepared nanoemulsion composition are shown in Table 2 below.

Examples 35 and 36 are not in the scope of the claims.

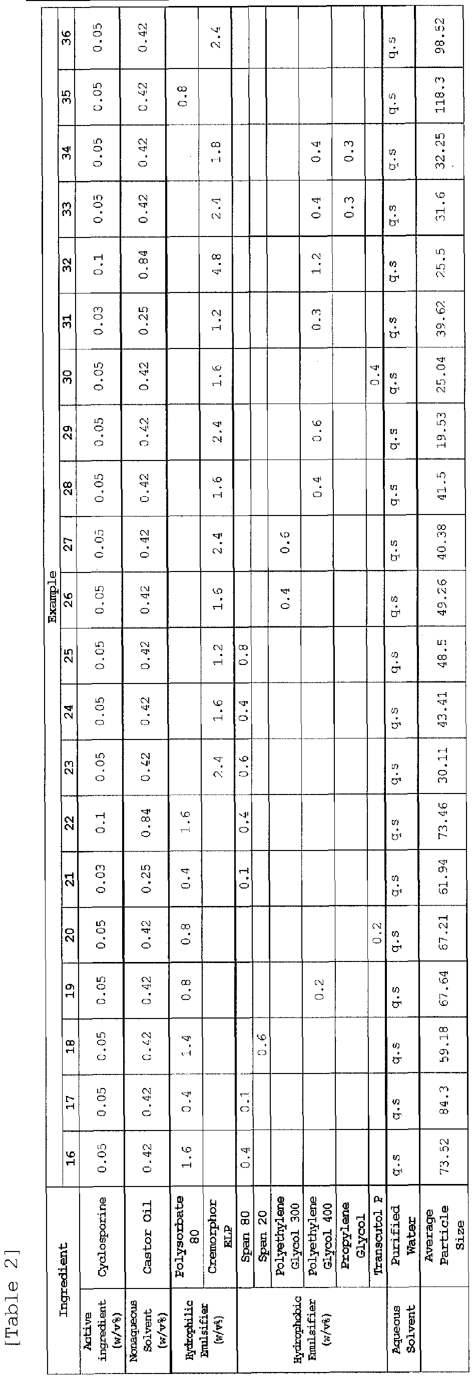

As shown in Table 2, it was confirmed that the average particle size of the nanoemulsion composition in Examples 16 to 34 were 100 nm or less, and that its particle size distribution was very narrow. Therefore, it was confirmed that the ophthalmic nanoemulsion composition having average particle size of 100 nm or less and narrow particle distribution can be prepared when mixing cyclosporine, castor oil, the hydrophilic emulsifier, the hydrophobic emulsifier and the aqueous solvent with the above constitution.

In Table 2, Examples 35 and 36 are the compositions prepared by only using the hydrophilic emulsifier except the hydrophobic emulsifier and accordingly an opaque emulsion having wide particle size distribution was formed, and especially, it was confirmed that Example 36 had very unsuitable particle distribution because its particle size distribution was wide as 17.02 - 382.5 nm. Therefore, it was confirmed that combination of the hydrophilic emulsifier and the hydrophobic emulsifier is preferred to prepare the nanoemulsion having a suitable particle size and particle distribution.

It is important to have a suitable particle size distribution to prepare the ophthalmic composition, and thus the particle size distribution of Example 33 which was prepared by the method of the present disclosure was compared to that of the commercially available Restasis™ eye drop.

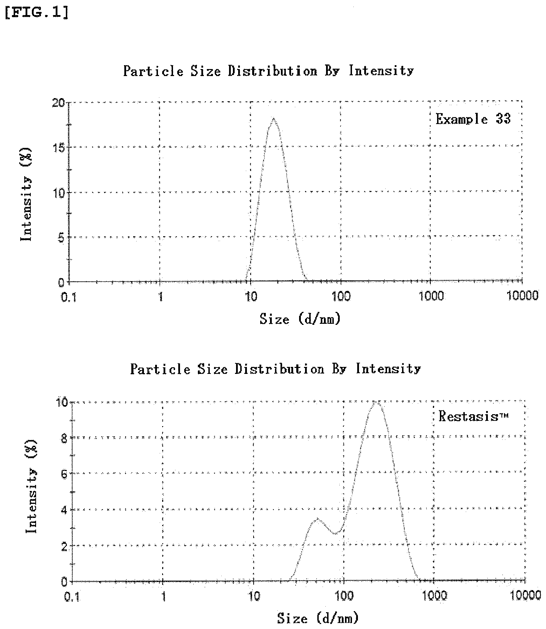

The result of the comparison is illustrated in FIG. 1.

As illustrated in FIG. 1, the composition according to the present disclosure (Example 33) had very narrow particle size distribution of 8.721 - 43.82 nm while that of the Restasis™ eye drop was very wide as 21.04 - 712.4 nm.

Namely, both that the nanoemulsion composition of the present disclosure can form more suitable composition for preparation of ophthalmic composition compared to the Restasis™ eye drop and that the sterilizing filtration by using 0.22 µm filter was available due to the maximum particle size of the nanoemulsion composition of the present disclosure as 220 nm or less, were confirmed.

### Experimental Example 3. Optimum content of the ingredients of the nanoemulsion composition

Formation of the nanoemulsion depending on variation of amount of each ingredient was confirmed to determine relative amount of the nonaqueous solvent, the hydrophilic emulsifier and the hydrophobic emulsifier which are suitable for preparation of cyclosporine ophthalmic nanoemulsion composition of the present disclosure.

### 3.1 Content of the nonaqueous solvent

Composition were prepared by using the same method explained in Experimental Example 1 with fixed contents of cyclosporine, the hydrophilic emulsifier and the hydrophobic emulsifier and varied amount of castor oil to 2.5, 3.0 and 3.5w/v%, and formation of the nanoemulsion was confirmed.

The result is shown in Table 3 below.

The compositions in the second and in the third column are not in the scope of the claims.

**[Table 3]**

| | | | |
|---|---|---|---|
| **Cyclosporine** | 0.05g | 0.05g | 0.05g |
| **Castor Oil** | 2.5g | 3.0 g | 3.5g |
| **Cremorphor ELP** | 5.0g | 5.0g | 5.0g |
| **PEG 400** | 2.0g | 2.0g | 2.0g |
| **Water for Injection** | q.s -> 100ml | q.s -> 100ml | q.s ->100ml |
| **Average Particle Size (nm)** | 45.75 | 105.0 | N/A |

As shown in Table 3, formation of the superior nanoemulsion having a particle size of 45.75nm was confirmed when comprising 2.5w/v% of castor oil based on the total amount of the composition, but the particle size was increased when increasing the amount of castor oil to 3.0 w/v% and the particle size could not be measured when increasing the amount of castor oil up to 3.5 w/v%. That is, it was confirmed that castor oil (i.e. the nonaqueous solvent) was preferred to be contained 8 times or more of cyclosporine (i.e. active ingredient) to prepare the nanoemulsion composition of the present disclosure, and the maximum amount of castor oil in a composition including hydrophilic surfactant up to 5w/v% was preferred to be 2.5 w/v% or less.

### 3.2 Amount of the emulsifier

The hydrophilic emulsifier and the hydrophobic emulsifier are necessary ingredients to form the nanoemulsion but these incur decline of sensation by ocular instillation when contained in excessively, and the nanoemulsion cannot be formed when insufficiently contained, and thus it is important to select an adequate amount of the hydrophilic emulsifier and the hydrophobic emulsifier. Therefore, the amount of the hydrophilic emulsifier and the hydrophobic emulsifier which are necessary for preparing the ophthalmic nanoemulsion composition of the present disclosure were confirmed through a comparative experiment. The amount of castor oil (i.e. the nonaqueous solvent) was set to be 8 times or more of the amount of cyclosporine, and preparation was conducted under same conditions to Experimental Example 1 with constituting the cremorphor ELP as the hydrophilic emulsifier and PEG 400 as the hydrophobic emulsifier. Each content and result of the nanoemulsion formation therefrom is shown in Table 4 below.

**[Table 4]**

| **Ingredient** | **1** | **2** | **3** | **4** | **5** |
|---|---|---|---|---|---|
| **Cyclosporine** | 0.05g | 0.05g | 0.05g | 0.05g | 0.03g |
| **Castor Oil** | 0.42g | 0.42g | 0.42g | 0.42g | 0.25g |
| **Cremorphor ELP** | 1. 8g | 1. 8g | 0.6g | 0.4g | 0.4g |
| **Polyethylene glycol 400** | 2.0g | 5.0g | 2.0g | 2.5g | 0.1g |
| **Water for Injection** | q.s -> 100ml | q.s -> 100ml | q.s -> 100ml | q.s -> 100ml | q.s -> 100ml |
| **Average Particle Size (nm)** | 22.96 | 27.25 | 74.53 | 127.9 | 48.67 |
| **Note** | - | - | - Wide Particle Distribution - Formation of Opaque Composition | - | - |

As shown in Table 4, the nanoemulsion having an average particle size of 100 nm or less was formed in every composition including cremorphor ELP (i.e. the hydrophilic emulsifier) 1.6 times or more of the amount of castor oil or including the PEG 400 (i.e. the hydrophobic emulsifier) in maximum of 3 times or less of the amount of the hydrophilic emulsifier, but confirmed that formation of the nanoemulsion in composition wherein the amount of cremorphor ELP is less than 1.6 times of the amount of castor oil and the amount of PEG 400 (i.e. the hydrophobic emulsifier) exceeded 3 times of the amount of cremorphor ELP (i.e. the hydrophilic emulsifier) was relatively difficult. Furthermore, the nanoemulsion was formed but could not maintain its phase due to low stability when contained the hydrophobic emulsifier less than 0.1 w/v% based on the total amount of the composition. Unpleasant feeling by ocular instillation gets worse when each amount of the hydrophilic emulsifier and the hydrophobic emulsifier was exceeded 5 w/v% based on the total amount of the composition, and thus it was confirmed that it is most preferable to include the hydrophilic emulsifier 1.6 times in minimum of the amount of castor oil (i.e. nonaqueous solvent) to 5 w/v% or less based on the total amount of the composition, and to include the hydrophobic emulsifier 0.1 w/v% based on the total amount of the composition to 3 times or less of the amount of the hydrophilic emulsifier and 5 w/v% or less based on the total amount of the composition to prepare the nanoemulsion composition having superior sensation by ocular instillation and stability.

### Experimental Example 4. Stabilizer-added nanoemulsion composition

As confirmed in Experimental Examples 1 through 3, the nanoemulsion composition having an average particle size of 100 nm or less can be prepared only by adding cyclosporine, the nonaqueous solvent, the hydrophilic emulsifier and the hydrophobic emulsifier. Furthermore, the stabilizer can be selectively added to prepare the ophthalmic nanoemulsion composition to improve stability because stable maintenance of particle size after preparation of the ophthalmic nanoemulsion composition is important. Therefore, it was confirmed whether the particle size of the nanoemulsion can be maintained when the stabilizer is added, and stability thereof.

### 4.1 Preparation of the stabilizer-added nanoemulsion composition and its average particle size

Specifically, the nanoemulsion compositions were prepared according to the contents stated in Table 5 below. The stabilizer and the isotonizing agent were hydrated in the aqueous solvent and its pH was adjusted to 7.2 by using NaOH and HCl. The oily phase was prepared by using the same method to Experimental Example 1 wherein cyclosporine, the nonaqueous solvent and the emulsifier of the contents stated in Table 5 were completely dissolved, it was put into the aqueous solvent, and it was stirred by the stirrer (Super-Nuova™ Multi-place, Thermo Scientific) under conditions of 400 - 500 rpm and room temperature. Single phase was formed through Self Nano-Emulsifying Drug Delivery system (SNEDDS) as Experimental Example 1. Particle size of the prepared nanoemulsion composition was measured in the same manner to Experimental Example 1. Each average particle size of the nanoemulsion was shown in Table 5 below.

**[Table 5]**

| **Ingredient** | | Example | | | | | | | |
|---|---|---|---|---|---|---|---|---|---|
| | | **37** | 38 | 39 | 40 | 41 | 42 | 43 | 44 |
| **Active ingredient (w/v%)** | **Cyclosporine** | 0.05 | 0.05 | 0.05 | 0.05 | 0.05 | 0.05 | 0.05 | 0.05 |
| **Nonaqueous Solvent (w/v%)** | **Castor Oil** | 0.42 | 0.42 | 0.42 | 0.42 | 0.42 | 0.42 | 0.42 | 0.42 |
| **Emulsifier (w/v%)** | **Cremorphor ELP** | 2.4 | 2.4 | 2.4 | 2.4 | 2.4 | 2.4 | 2.4 | 2.4 |
| | **Polyethylene glycol 400** | 0.6 | 0.6 | 0.6 | 0.6 | 0.6 | 0.6 | 0.6 | 0.6 |
| **Stabilizer (w/v%)** | **Sodium Hyaluronate** | 0.1 | - | - | - | - | - | - | - |
| | **Sodium Alginate** | - | 0.2 | - | - | - | - | - | - |
| | **Hydroxypropylmethylcellulose** | - | - | 0.5 | - | - | - | - | - |
| | **Polyvinylpyrrolidone** | - | - | - | 1.2 | - | - | - | - |
| | **Hydroxyethylcellulose** | - | - | - | - | 0.5 | - | - | - |
| | **Polyvinyl alcohol** | - | - | - | - | - | 1.4 | - | - |
| | **Xanthan Gum** | - | - | - | - | - | - | 0.2 | - |
| | **Sodium Carboxymethyl Cellulose** | - | - | - | - | - | - | - | 0.5 |
| **Isotonizing Agent (v/v%)** | **Glycerol** | 2.2 | 2.2 | 2.2 | 2.2 | 2.2 | 2.2 | 2.2 | 2.2 |
| **pH adjusting agent** | **NaOH, HCl** | q.s →7.2 | q.s →1.2 | q.s →7.2 | q.s →7.2 | q.s →7.2 | q.s →7.2 | q.s →7.2 | q.s →7.2 |
| **Aqueous Solvent** | **Purified Water(ml)** | q.s | q.s | q.s | q.s | q.s | q.s | q.s | q.s |
| | **Average Particle Size** | 28.15 | 25.62 | 61.75 | 40.41 | 60.33 | 41.03 | 33.93 | 33.15 |

As shown in Table 5, the nanoemulsion composition having an average size of 100 nm or less was formed even with the addition of the stabilizer and it can be confirmed that its particle size distribution of the prepared composition was narrow. Accordingly, it was confirmed that the preferred particle size and particle size distribution of the present disclosure can be maintained even if additionally comprising the stabilizer.

### 4.2 Evaluation of thermal stability of the nanoemulsion composition

The nanoemulsion compositions were prepared with the same content stated in Table 6 below by using the same method to Experimental Example 4(1). To evaluate physicochemical stabilities of the nanoemulsion composition, the amount of cyclosporine and average particle size of the nanoemulsion composition were analyzed while storing the nanoemulsion composition for 2 weeks under high temperature of 70±2°C. The amount of cyclosporine was measured by chromatography, the ACQUITY Ultra Pressure Liquid Chromatography (UPLC) system (Waters Asia Ltd., 396 Alexandra Road #04-06 BP Tower, Singapore 119954), under analysis condition stated in Table 7 below.

**[Table 6]**

| Ingredient | | **Example** | | | | | | | | | | | | | | | | |
|---|---|---|---|---|---|---|---|---|---|---|---|---|---|---|---|---|---|---|
| | | **45** | **46** | **47** | **48** | **49** | **50** | **51** | **52** | **53** | **54** | **55** | **56** | **57** | **58** | **59** | **60** | **61** |
| **Active ingredient (w/v%)** | **Cyclosporine** | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 |
| **Nonaqueous Solvent (w/v%)** | **Castor Oil** | 0.42 | 0.42 | 0.42 | 0.42 | 0.42 | 0.42 | 0.42 | 0.42 | 0.42 | 0.42 | 0.42 | 0.42 | 0.42 | 0.42 | 0.42 | 0.42 | 0.42 |
| **Emulsifier (w/v%)** | **Cremorphor ELP** | 2.4 | 2.4 | 2.4 | 2.4 | 2.4 | 2.4 | 2.4 | 2.4 | 2.4 | 2.4 | 2.4 | 2.4 | 1.8 | 1.8 | 1.8 | 1.8 | 1.8 |
| | **Polyethylene glycol 400** | 0.6 | 0.6 | 0.6 | 0.6 | 0.6 | 0.6 | 0.4 | 0.4 | 0.4 | 0.4 | 0.4 | 0.4 | 0.4 | 0.4 | 0.4 | 0.4 | 0.4 |
| | **Propylene Glycol** | | | | | | | 0.3 | 0.3 | 0.3 | 0.3 | 0.3 | 0.3 | 0.3 | 0.3 | 0.3 | 0.3 | 0.3 |
| **Stabilizer (w/v%)** | **Sodium Hyaluronate** | | 0.1 | | | | | | 0.1 | | | | | | | | | |
| | **Polyvinylpyrrolidone** | | | 1.8 | | | | | | 1.8 | | | | | 1.8 | | | |
| | **Sodium Alginate** | | | | 0.2 | | | | | | 0.2 | | | | | | | |
| | **Sodium Carboxymethyl Cellulose** | | | | | 0.5 | | | | | | 0.5 | | | | 0.5 | 0.2 | |
| | **Polyvinylalchol** | | | | | | 1.4 | | | | | | 1.4 | | | | | |
| | **Xanthan Gum** | | | | | | | | | | | | | | 0.1 | 0.1 | | 0.2 |
| **Isotonizing Agent (v/v%)** | **Glycerol** | 2 | 2 | 2 | 2 | 2 | 2 | 1.7 | 1.7 | 1.7 | 1.7 | 1.7 | 1.7 | 1.7 | 1.7 | 1.7 | 1.7 | 1.7 |
| **pH adjusting agent** | **NaOH, HCl** | q.s →7.2 | q.s →7.2 | q.s →7.2 | q.s →7.2 | q.s →7.2 | q.s →7.2 | q.s →7.2 | q.s →7.2 | q.s →7.2 | q.s →7.2 | q.s →7.2 | q.s →7.2 | q.s →7.2 | q.s →7.2 | q.s →7.2 | q.s →7.2 | q.s→7.2 |
| **Aqueous Solvent** | **Purified Water(ml)** | q.s | q.s | q.s | q.s | q.s | q.s | q.s | q.s | q.s | q.s | q.s | q.s | q.s | q.s | q.s | q.s | q.s |

**[Table 7]**

| Analytical Conditions of the UPLC | |
|---|---|
| **Column** | UPLC column 1HSS T3.8 µm 2.1 X 100 mm |
| **Wavelength of UV detector** | 210 nm |
| **Column Temperature** | 75 °C |
| **Sample Temperature** | 25 °C |
| **Flow Rate** | 0.250 ml/min |
| **Injection Volume** | 5 µl |
| **Run Time** | 20 min |
| **Mobile Phase** | Acetonitrile(ACN) : water = 68:32 |

Measurement results of content(%)of cyclosporine and average particle size(nm) in the nanoemulsion composition, which were stored for 2 weeks under high temperature, are shown in Table 8 below.

**[Table 8]**

| **Item** | **Duration (week)** | **Example** | | | | | | | | | | | | | | | | |
|---|---|---|---|---|---|---|---|---|---|---|---|---|---|---|---|---|---|---|
| | | **45** | **46** | **47** | **48** | **49** | **50** | **51** | **52** | **53** | **54** | **55** | **56** | **57** | **58** | **59** | **60** | **61** |
| **Content** | 0 | 101.97 | 102 | 101.66 | 98.32 | 99.91 | 98.32 | 101.87 | 100.6 | 100.36 | 101.7 | 100.91 | 101.64 | 100.76 | 101.4 | 100.35 | 102.24 | 102.24 |
| | 1 | 97.17 | 103.8 | 103.55 | 100.7 | 103.3 | 101.1 | 97.85 | 98.02 | 97.08 | 100.1 | 99.45 | 99.93 | 97.03 | 99.58 | 99.6 | 98.7 | 98.7 |
| | 2 | 92.96 | 99.93 | 102.47 | 96.83 | 94.64 | 96.83 | 90.32 | 91.86 | 98.73 | 96.93 | 98.18 | 94.64 | 91.15 | 98.04 | 100.5 | 99.26 | 99.26 |
| **Size** | 0 | 17.85 | 30.59 | 25.41 | 24.06 | 33.15 | 36.11 | 18.53 | 32.64 | 30.14 | 23.19 | 32.44 | 37.8 | 25.04 | 41.85 | 38.38 | 48.13 | 36.88 |
| | 1 | 30.12 | 27.9 | 28.26 | 24.34 | 35.15 | 50.85 | 21.02 | 34.57 | 31.66 | 23.55 | 34.54 | 46.91 | 31.81 | 42.39 | 35.98 | 44.33 | 34.08 |
| | 2 | 50.04 | 23.92 | 29.86 | 23.63 | 33.66 | 50.8 | 53.06 | 34.29 | 38.84 | 27.79 | 35.31 | 51.53 | 54.98 | 42.5 | 36.27 | 44.19 | 38.62 |

As shown in Table 8, the content of cyclosporine in Examples 45 to 61 were all suitable for assay criteria (90 - 110%) and maintained the initial average particle size of 100 nm or less under high temperature of 70±2°C even after 2 weeks. The contents of cyclosporine in Examples 45, 51 and 57 wherein the stabilizer was not added were all equal to or greater than 90% and confirmed that the particle size was maintained as 100 nm or less, but in the rest of the Examples wherein the stabilizer was added, it was confirmed that the content of cyclosporine decreasing rate and the particle size variation were very low. Therefore, it was confirmed that the nanoemulsion composition of the present disclosure can maintain its physicochemical stabilities under high temperature, and especially, the physicochemical stabilities could be further improved when the stabilizer is added.

### Experimental Example 5. Evaluation of stability of the nanoemulsion composition

### 5.1 Evaluation of long-term stability of the nanoemulsion composition

In order to use as the ophthalmic composition, long-term physicochemical stability should be secured under accelerated storage condition (40±2°C, 25%RH or less) which is a condition that can confirm the stability of sample as a 6-month short-term stability data for authorization before long-term physicochemical stability test under room temperature (25±2°C, 40±5%RH) and 24 to 36 months. Therefore, the nanoemulsion compositions of Examples 45 to 61 were stored for 6 months under room temperature and the accelerated storage condition, and the content of cyclosporine and particle size of the nanoemulsion composition were measured at 0^{th}, 3^{rd} and 6^{th} month. A method of measuring content of cyclosporine and particle size was equivalent to Experimental Example 3.

The results of the above are shown in Tables 9 and 10 below.

As shown in Tables 9 and 10, the nanoemulsion compositions of Examples 45 to 61 were confirmed as very stable under the room temperature condition in Table 9 and the accelerated condition in Table 10 due to low variation of content and particle size in long-term.

**[Table 9]**

| **Item** | **Duration (month)** | **Example** | | | | | | | | | | | | | | | | |
|---|---|---|---|---|---|---|---|---|---|---|---|---|---|---|---|---|---|---|
| | | **45** | **46** | **47** | **48** | **49** | **50** | **51** | **52** | **53** | **54** | **55** | **56** | **57** | **58** | **59** | **60** | **61** |
| **Content** | **0** | 101.97 | 102.0 | 101.66 | 98.32 | 99.91 | 98.32 | 101.87 | 100.6 | 100.36 | 101.7 | 100.91 | 101.64 | 100.76 | 101.4 | 100.35 | 102.24 | 102.24 |
| | **3** | 96.08 | 103.43 | 101.37 | 99.45 | 100.63 | 100.56 | 96.24 | 102.57 | 101.36 | 100.64 | 101.17 | 102.74 | 96.07 | 103.89 | 103.60 | 104.26 | 102.68 |
| | **6** | 96.33 | 103.26 | 101.49 | 101.85 | 104.24 | 103.62 | 98.84 | 104.22 | 102.57 | 101.08 | 104.85 | 102.23 | 99.37 | 103.51 | 104.58 | 105.97 | 100.18 |
| **Size** | **0** | 17.85 | 30.59 | 25.41 | 24.06 | 33.15 | 36.11 | 18.53 | 32.64 | 30.14 | 23.19 | 32.44 | 37.8 | 25.04 | 41.85 | 38.38 | 48.13 | 36.88 |
| | **3** | 21.08 | 31.44 | 26.97 | 24.24 | 35.44 | 45.79 | 18.62 | 32.96 | 31.20 | 25.77 | 32.75 | 44.30 | 19.19 | 41.90 | 50.55 | 36.68 | 39.26 |
| | **6** | 20.88 | 31.14 | 26.52 | 24.20 | 35.25 | 45.66 | 18.63 | 33.05 | 31.20 | 22.16 | 32.36 | 44.46 | 19.64 | 48.30 | 52.81 | 36.47 | 41.18 |

**[Table 10]**

| **Item** | **Duration (month)** | **Example** | | | | | | | | | | | | | | | | |
|---|---|---|---|---|---|---|---|---|---|---|---|---|---|---|---|---|---|---|
| | | **45** | **46** | **47** | **48** | **49** | **50** | **51** | **52** | **53** | **54** | **55** | **56** | **57** | **58** | **59** | **60** | **61** |
| **Content** | **0** | 101.97 | 102.0 | 101.66 | 98.32 | 99.91 | 98.32 | 101.87 | 100.6 | 100.36 | 101.7 | 100.91 | 101.64 | 100.76 | 101.4 | 100.35 | 102.24 | 102.24 |
| | **3** | 102.27 | 99.79 | 100.47 | 101.39 | 103.49 | 101.97 | 105.02 | 96.70 | 100.28 | 99.10 | 105.87 | 106.93 | 106.18 | 99.27 | 100.60 | 104.49 | 104.62 |
| | **6** | 102.86 | 100.87 | 102.21 | 102.01 | 101.95 | 102.31 | 102.01 | 102.54 | 101.87 | 99.95 | 102.18 | 103.35 | 107.29 | 102.39 | 104.02 | 103.43 | 99.84 |
| **Size** | **0** | 17.85 | 30.59 | 25.41 | 24.06 | 33.15 | 36.11 | 18.53 | 32.64 | 30.14 | 23.19 | 32.44 | 37.8 | 25.04 | 41.85 | 38.38 | 48.13 | 36.88 |
| | **3** | 31.99 | 41.36 | 41.45 | 34.02 | 54.54 | 74.07 | 29.10 | 48.86 | 47.68 | 31.80 | 60.35 | 71.25 | 23.77 | 44.96 | 58.16 | 41.02 | 40.46 |
| | **6** | 31.47 | 40.70 | 40.88 | 33.80 | 54.79 | 73.64 | 29.20 | 49.81 | 47.56 | 31.62 | 58.92 | 71.62 | 23.78 | 45.39 | 55.42 | 41.15 | 43.02 |

### 5.2 Evaluation of a distribution stability of the nanoemulsion composition

In order to evaluate the long-term stability of the nanoemulsion composition compare to an emulsion and a suspension, dispersion stability test was conducted by using the Turbiscan (Turbiscan Ageing Station, Formulaction, France) in accordance with the manufacturer's manual. The constitutions of the nanoemulsion composition, the emulsion and the suspension used in the test were shown in Table 11 below and the nanoemulsion composition were prepared by using the same method to Experimental Example 4(1). The preparation method of the emulsion follows: The stabilizer and the isotonizing agent of Table 11 were hydrated in the aqueous solvent and its pH was adjusted to 7.2 by using NaOH and HCl. The oily phase was prepared by completely dissolving cyclosporine, the nonaqueous solvent and the emulsifier in content stated in Table 11 *via* the same method to Experimental Example 1. The prepared oily phase was put in the aqueous solvent and stirred under 400 - 500 rpm and room temperature by using the stirrer (Super-Nuova™ Multi-place, Thermo Scientific). It was emulsified by using the high speed stirrer (Homomixer T-Basic 25, IKA™) under speed range of 9000 - 17500 rpm, and the emulsion was prepared through a bubble removal process and a cooling process to a room temperature. The suspension of this example was prepared through following methods: The stabilizer and the isotonizing agent of Table 11 were hydrated in the aqueous solvent and its pH was adjusted to 7.2 by using NaOH and HCl. Cyclosporine and the emulsifier in content stated in Table 11 were put into the aqueous solvent and stirred under 400 - 500 rpm and room temperature by using the stirrer (Super-Nuova™ Multi-place, Thermo Scientific) for 10 minutes. It was dispersed by using the high speed stirrer (Homomixer T-Basic 25, IKA™) under speed range of 9000 - 13500 rpm, and the suspension was prepared through the bubble removal process and the cooling process to a room temperature.

**[Table 11]**

| **Ingredient (w/v%)** | | **Example** | | **Emulsion** | **Suspension** |
|---|---|---|---|---|---|
| | | 62 | 63 | | |
| **Active ingredient** | **Cyclosporine** | 0.05 | 0.05 | 0.05 | 0.05 |
| **Nonaqueous Solvent** | **Castor Oil** | 0.42 | 0.42 | 1.26 | - |
| **Emulsifier** | **Cremorphor ELP** | 1.8 | 1.8 | - | - |
| | **Polysorbate 80** | - | - | 1.0 | 1.0 |
| | **polyethylene glycol 400** | 0.4 | 0.4 | - | - |
| | **Propylene Glycol** | 0.3 | 0.3 | - | - |
| **Stabilizer** | **Sodium Carboxymethyl Cellulose** | 0.1 | 1.0 | - | 0.5 |
| | **Xanthan Gum** | 0.1 | 0.6 | 0.6 | 0.3 |
| **Buffering Agent** | **Boric Acid** | 0.2 | 1.0 | - | - |
| **Isotonizing Agent** | **Glycerol** | 1.7 | 0.3 | 2.2 | 2.2 |
| **pH adjusting agent** | **NaOH** | q. s →7.2 | q. s →7.2 | q. s →7.2 | q. s →7.2 |
| **Aqueous Solvent** | **Purified Water(ml)** | q. s | q. s | q. s | q. s |

The variation of the dispersion stability according to time under certain temperature can be measured when using the Turbiscan, and thus the dispersion stability was measured at 50°C in conformity with the manufacturer's manual. In detail, the nanoemulsion composition, the emulsion and the suspension were shaken and injected to the Turbiscan, respectively, it was rested under temperature of 50°C for 48 hours, and variation pattern of each sample according to obtained Turbiscan Stability Index (TSI) which was measured by the Turbiscan was observed.

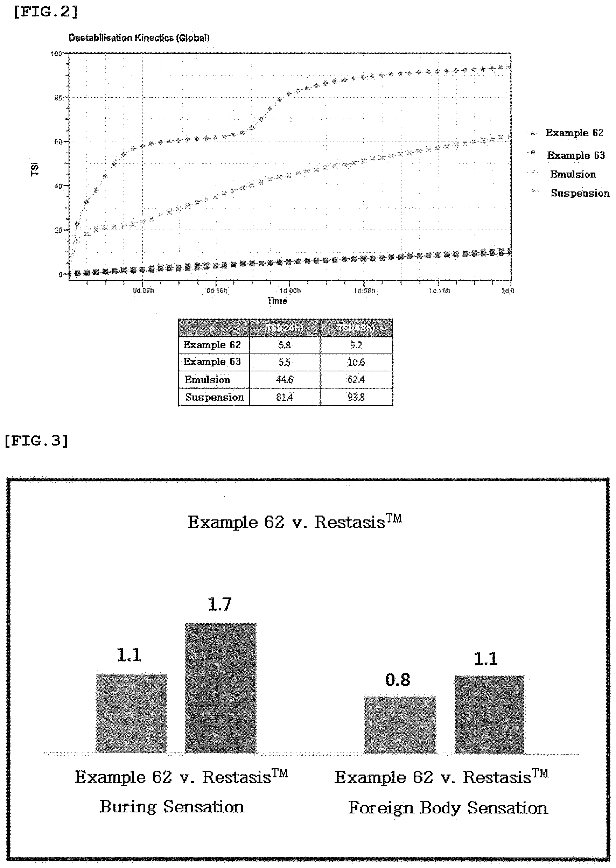

TSI results of Examples 62 and 63 are illustrated in FIG. 2.

As illustrated in FIG. 2, TSI value of the nanoemulsion composition after 48 hours were 9.2(Example 62) and 10.6(Example 63). These TSI values were very low compared to that of the emulsion (i.e. 62.4) and the suspension (i.e. 93.8) which were measured under the same conditions. Accordingly, it was confirmed that the stability of the prepared nanoemulsion composition was markedly superior to the existing emulsions or suspensions.

### Experimental Example 6. In vitro drug release test of the nanoemulsion composition

To confirm whether the prepared nanoemulsion composition releases cyclosporine A (i.e. active ingredient) with an appropriate rate, *in vitro* cellulose membrane-release assay which evaluates release of drugs was conducted. Constitutions of the nanoemulsion composition for this assay was identical to that of Examples 62 and 63 in Experimental Example 5(2), and prepared by the same method to Experimental Example 4(1). The commercially available Restasis™ was used as a control drug.

In detail, the membrane (100 kDa Cellulose Ester Membrane) was cut to the appropriate size and soaked over an hour in a medium solution (70% MeOH + 30% Balanced Salt Solution (BSS)). The prepared medium in beakers of the same size was dispensed as 100 ml, and magnetic bars in the same shape and size were placed in each beaker. The opposite ends of the membranes were folded and sealed with sealing bars, and the nanoemulsion composition and the control drug were put inside of the membranes respectively, and inserted the membranes containing the drug into the beakers at the same height while the membranes were completely immersed in the beakers but not to contact with the magnetic bars. Stirring was conducted at the same time under identical speed of 150 rpm. The samples were obtained at appropriate time intervals and measured the amount of cyclosporine by the content test through the UPLC in the same manner to Experimental Example 4(2).

As a result of the drug release test, it was confirmed that the nanoemulsion compositions of Examples 62 and 63 commonly maintained constant drug concentration after about 5 hours from the start of the test and reached to Steady State Concentration (Css). Accordingly, the endpoint of the drug release test was set at 6 hours after the start of the test. The concentration of the drug in Example 62 was confirmed as 52.0% through measurement after 5 hours, and it was shown as 54.2% after 6 hours (i.e. the endpoint). These drug concentration was very similar to 55.3% (5 hours) and 56.3% (6 hours) which were the concentration of the control drug (i.e. Restasis™), and thus it was confirmed that the prepared nanoemulsion composition was a suitable composition for eye drop.

### Experimental Example 7. Evaluation of ocular irritation of the nanoemulsion composition

Ocular irritation evaluation was conducted for testing sensation by ocular instillation by using the composition of Example 62 within the prepared nanomeulsion compositions. 30 µl of the nanoemulsion composition of Example 62 was administered to both eyes of 40 healthy adults, and burning sensation and foreign body sensation of drop into the eyes of each person were evaluated and scored after 10 minutes according to the scales in Table 12 below. Restasis™ was used as the control drug.

**[Table 12]**

| **Scale** | **Burning Sensation** | **Foreign Body Sensation** |
|---|---|---|
| **0** | No pruritus, very soft | No foreign body sensation, no viscous sensation |
| **1-2** | Little pruritus(stinging) | Little foreign body sensation and viscosity |
| **3-4** | Pruritus(stinging) | Continuing foreign body sensation accompanying little pain, strong sensation of viscosity |
| **5** | Immediate sensation of irritation and very uncomfortable | Long-lasting foreign body sensation and viscosity with strong pain |

The results were shown in Table 13 and FIG. 3.

**[Table 13]**

| Subject | Burning Sensation | | Foreign Body Sensation | |
|---|---|---|---|---|
| | Example 62 | Restasis™ | Example 62 | Restasis™ |
| 1 | 0 | 4 | 0 | 4 |
| 2 | 1 | 3 | 1 | 3 |
| 3 | 1 | 0 | 1 | 0 |
| 4 | 2 | 2 | 0 | 2 |
| 5 | 2 | 0 | 1 | 0 |
| 6 | 2 | 4 | 1 | 3 |
| 7 | 3 | 2 | 0 | 0 |
| 8 | 2 | 0 | 1.5 | 1 |
| 9 | 2 | 1 | 0 | 0 |
| 10 | 1 | 0 | 0 | 0 |
| 11 | 0 | 0 | 1 | 1 |
| 12 | 1 | 3 | 0 | 0 |
| 13 | 0 | 2 | 0 | 0 |
| 14 | 0 | 1 | 0 | 0 |
| 15 | 2 | 1 | 0 | 2 |
| 16 | 0 | 0 | 2 | 2 |
| 17 | 2 | 1 | 1 | 1 |
| 18 | 0 | 3 | 0 | 0 |
| 19 | 1 | 3 | 1 | 2 |
| 20 | 0 | 0 | 0 | 0 |
| 21 | 3 | 2 | 3 | 1 |
| 22 | 1 | 3 | 3 | 1 |
| 23 | 1 | 0 | 2 | 0 |
| 24 | 0 | 3 | 0 | 0 |
| 25 | 0 | 1 | 0 | 0 |
| 26 | 1 | 4 | 0 | 4 |
| 27 | 0 | 0 | 2 | 1 |
| 28 | 3 | 4 | 1 | 1 |
| 29 | 0 | 0 | 1 | 0 |
| 30 | 0 | 2 | 0 | 2 |
| 31 | 4.5 | 3.5 | 3 | 3 |
| 32 | 0 | 1 | 0 | 1 |
| 33 | 0 | 1 | 6 | 0 |
| 34 | 3 | 4 | 2 | 1 |
| 35 | 0 | 2 | 1 | 1 |
| 36 | 1 | 2 | 0 | 1 |
| 37 | 3 | 2 | 0 | 1 |
| 38 | 0 | 2 | 1 | 1 |
| 39 | 0 | 1 | 0 | 1 |
| 40 | 0 | 2 | 1 | 1 |
| Total | 42.5 | 69.5 | 30.5 | 42 |
| Average | 1.1 | 1.7 | 0.8 | 1.1 |

As shown in Table 13 and FIG. 3, the ophthalmic nanoemulsion of Example 62 exhibited average scores 1.1 of burning sensation and 0.8 of foreign body sensation which were lower than that of control drug Restasis™ (i.e., 1.7 of burning sensation and 1.1 of foreign body sensation). Namely, it was confirmed that the ophthalmic nanoemulsion was a composition that shows more improved sensation by ocular instillation compared to the existing Restasis™.

### [Industrial Applicability]

The ophthalmic nanoemulsion composition according to the present disclosure is characterized in having average particle size of is 200 nm or less, preferably 100 nm or less and having a narrow particle distribution, and thus sterilizing filtration is available, stability can be improved and has superior effect in clinical enhancement of foreign body sensation and visual disturbance. Therefore, the ophthalmic nanoemulsion composition of the present disclosure can be effectively used as an ophthalmic composition.

## Claims

1. An ophthalmic nanoemulsion composition comprising:
cyclosporine in the amount of 0.02 - 0.3 w/v% based on the total amount of the composition;
castor oil in the amount of 8 times or more of the amount of the cyclosporine to 2.5 w/v% or less based on the total amount of the composition;
one or more hydrophilic emulsifiers selected from a group consisting of polyoxyl 35 castor oil, polyoxyethylene sorbitan fatty acid esters and polyoxyethylene fatty acid esters;
one or more hydrophobic emulsifiers selected from a group consisting of sorbitan fatty acid esters, glycerin fatty acid esters, diethylene glycol monoethyl ether, polyethylene glycols, propylene glycol and propylene glycol esters of fatty acids; and
an aqueous solvent.

2. The ophthalmic nanoemulsion composition according to claim 1, wherein the hydrophilic emulsifier is the polyoxyl 35 castor oil and
the amount of hydrophilic emulsifier is 1.6 times or more of the amount of the castor oil to 5.0 w/v% or less based on the total amount of the composition.

3. The ophthalmic nanoemulsion composition according to claim 2, wherein the hydrophobic emulsifier is at least one selected from a group consisting of the polyethylene glycols, the propylene glycol and the diethylene glycol monoethyl ether, and
the amount of the hydrophobic emulsifier is 0.1 w/v% or more based on the total amount of the composition to 3 times or less based on the amount of the hydrophilic emulsifier.

4. The ophthalmic nanoemulsion composition according to claim 1, wherein:
the hydrophilic emulsifier is the polyoxyl 35 castor oil and the amount of the hydrophilic emulsifier is 12.8 times or more of the amount of the cyclosporine to 5.0 w/v% based on the total amount of the composition; and
the hydrophobic emulsifier is at least one selected from a group consisting of the polyethylene glycols, the propylene glycol and the diethylene glycol monoethyl ether, and the amount of the hydrophobic emulsifier is 0.1 w/v% to 5.0 w/v% based on the total amount of the composition.

5. The ophthalmic nanoemulsion composition according to claim 1, wherein the ophthalmic nanoemulsion composition further comprises one or more stabilizers selected from a group consisting of carboxymethyl cellulose (CMC), hydroxypropyl methylcellulose (HPMC), hydroxyethylcellulose (HEC), polyvinyl alcohol (PVA), polyvinylpyrrolidone (PVP), carbomer, gellan gum, xanthan gum, hyaluronic acid (HA), sodium hyaluronate, sodium alginate and dextran.

6. The ophthalmic nanoemulsion composition according to claim 5, wherein the amount of the stabilizer is 0.01 - 2.0 w/v% based on the total amount of the composition.

## Patentansprüche

1. Ophthalmische Nanoemulsionszusammensetzung, umfassend:
Cyclosporin in der Menge von 0,02 bis 0,3 % Gew./Vol., bezogen auf die Gesamtmenge der Zusammensetzung;
Castoröl in der Menge der 8-fachen Menge des Cyclosporins oder mehr bis zu 2,5 % Gew./Vol. oder weniger, bezogen auf die Gesamtmenge der Zusammensetzung;
einen oder mehrere hydrophile Emulgatoren ausgewählt aus einer Gruppe bestehend aus Polyoxyl 35-Castoröl, Polyoxyethylensorbitanfettsäureestern und Polyoxyethylenfettsäureestern;
einen oder mehreren hydrophobe Emulgatoren ausgewählt aus einer Gruppe bestehend aus Sorbitanfettsäureestern, Glycerinfettsäureestern, Diethylenglykolmonoethylether,
Polyethylenglykolen, Propylenglykol und Propylenglykolestern von Fettsäuren; und
ein wässriges Lösungsmittel.

2. Ophthalmische Nanoemulsionszusammensetzung nach Anspruch 1, wobei der hydrophile Emulgator das Polyoxyl 35-Castoröl ist; und
die Menge des hydrophilen Emulgators das 1,6-fache der Menge des Castoröls oder mehr bis zu 5,0 % Gew./Vol. oder weniger ist, bezogen auf die Gesamtmenge der Zusammensetzung.

3. Ophthalmische Nanoemulsionszusammensetzung nach Anspruch 2, wobei der hydrophobe Emulgator mindestens einer ausgewählt aus einer Gruppe bestehend aus den Polyethylenglykolen, dem Propylenglykol und dem Diethylenglykolmonoethylether ist, und
die Menge des hydrophoben Emulgators 0,1 % Gew./Vol. oder mehr, bezogen auf die Gesamtmenge der Zusammensetzung, bis zu der 3-fachen Menge oder weniger ist, bezogen auf die Menge des hydrophilen Emulgators.

4. Ophthalmische Nanoemulsionszusammensetzung nach Anspruch 1, wobei
der hydrophile Emulgator das Polyoxyl 35-Castoröl ist, und die Menge des hydrophilen Emulgators das 12,8-fache der Menge des Cyclosporins oder mehr bis zu 5,0 % Gew./Vol. ist, bezogen auf die Gesamtmenge der Zusammensetzung; und
der hydrophobe Emulgator mindestens einer ausgewählt aus einer Gruppe bestehend aus den Polyethylenglykolen, dem Propylenglykol und dem Diethylenglykolmonoethylether ist, und die Menge des hydrophoben Emulgators 0,1 % Gew./Vol. bis 5,0 % Gew./Vol. ist, bezogen auf die Gesamtmenge der Zusammensetzung.

5. Ophthalmische Nanoemulsionszusammensetzung nach Anspruch 1, wobei die ophthalmische Nanoemulsionszusammensetzung ferner einen oder mehrere Stabilisatoren ausgewählt aus einer Gruppe bestehend aus Carboxymethylcellulose (CMC), Hydroxypropylmethylcellulose (HPMC), Hydroxyethylcellulose (HEC), Polyvinylalkohol (PVA), Polyvinylpyrrolidon (PVP), Carbomer, Gellangummi, Xanthangummi, Hyaluronsäure (HA), Natriumhyaluronat, Natriumalginat und Dextran umfasst.

6. Ophthalmische Nanoemulsionszusammensetzung nach Anspruch 5, wobei die Menge des Stabilisators 0,01 bis 2,0 % Gew./Vol. beträgt, bezogen auf die Gesamtmenge der Zusammensetzung.

## Revendications

1. Composition ophtalmique en nano-émulsion comprenant : de la cyclosporine dans une proportion comprise entre 0,02 et 0,3 % en masse par volume par rapport à la quantité totale de la composition ;
de l'huile de ricin dans une proportion comprise entre 8 fois ou plus la proportion de la cyclosporine et 2,5 % en masse par volume ou moins par rapport à la quantité totale de la composition ;
un ou plusieurs émulsifiants hydrophiles choisis dans un groupe constitué par de l'huile de ricin polyoxyl 35, des esters d'acide gras de polyoxyéthylène sorbitane et des esters d'acide gras de polyoxyéthylène ; un ou plusieurs émulsifiants hydrophobes choisis dans un groupe constitué par les esters d'acide gras de sorbitane, les esters d'acide gras de glycérine, l'éther monoéthylique de diéthylène glycol, les polyéthylène glycols, le propylène glycol et les esters de propylène glycol d'acides gras ; et
un solvant aqueux.

2. Composition ophtalmique en nano-émulsion selon la revendication 1, où l'émulsifiant hydrophile est l'huile de ricin de polyoxyl 35 et
la proportion de l'émulsifiant hydrophile est comprise entre 1,6 fois ou plus la proportion de l'huile de ricin et 5,0 % en masse par volume ou moins par rapport à la quantité totale de la composition.

3. Composition ophtalmique en nano-émulsion selon la revendication 2, où l'émulsifiant hydrophobe est au moins l'un de ceux qui sont choisis dans un groupe constitué par les polyéthylène glycols, le propylène glycol et l'éther monoéthylique de diéthylène glycol, et
la proportion de l'émulsifiant hydrophobe est compris entre 0,1 % en masse par volume ou plus par rapport à la quantité totale de la composition et 3 fois ou moins la proportion de l'émulsifiant hydrophile.

4. Composition ophtalmique en nano-émulsion selon la revendication 1, où :
l'émulsifiant hydrophile est l'huile de ricin polyoxyl 35 et la proportion de l'émulsifiant hydrophile est comprise entre 12,8 fois la proportion de la cyclosporine et 5,0 % en masse par volume par rapport à la masse totale de la composition ; et
l'émulsifiant hydrophobe est au moins l'un de ceux qui sont choisis dans un groupe constitué par les polyéthylène glycols, le propylène glycol et l'éther monoéthylique de diéthylène glycol, et la proportion de l'émulsifiant hydrophobe est comprise entre 0,1 % en masse par volume et 5,0 % en masse par volume par rapport à la quantité totale de la composition.

5. Composition ophtalmique en nano-émulsion selon la revendication 1, où la composition ophtalmique en nano-émulsion comprend en outre un ou plusieurs stabilisants choisis dans un groupe constitué par les suivants : carboxyméthylcellulose (CMC), hydroxypropylméthylcellulose (HPMC), hydroxyéthylcellulose (HEC), alcool polyvinylique (PVA), polyvinylpyrrolidone (PVP), carbomère, gomme gellane, gomme xanthane, acide hyaluronique (HA), hyaluronate de sodium, alginate de sodium et dextrane.

6. Composition ophtalmique en nano-émulsion selon la revendication 5, où la proportion du stabilisant est de 0,01 à 2,0 % en masse par volume par rapport à la quantité totale de la composition.
